# EUROPÄISCHE PATENTANMELDUNG

(11) **EP 3 461 344 A2**
(43) Veröffentlichungstag der Anmeldung: **03.04.2019**
(21) Anmeldenummer: 18191001.9
(22) Anmeldetag: 19.06.2015
(51) Int. Cl.: A23L 3/04, B01D 21/00, A61L 2/10, A61L 2/22

(54) **TEMPERIERUNGSSYSTEM MIT REINIGUNG DER PROZESSFLÜSSIGKEIT**

(30) Priorität: 24.06.2014 DE 102014108798
(62) Teilanmeldung aus: 15172842.5
(71) Anmelder: KRONES AG, 93073 Neutraubling (DE)
(72) Erfinder: Muenzer, Jan, 93073 Neutraubling (DE)
(74) Vertreter: Grünecker Patent- und Rechtsanwälte PartG mbB

(57) **Zusammenfassung**

Temperierungssystem mit Reinigung der Prozessflüssigkeit, mit einer Zu- und Abführfördereinrichtung für Behälter; mindestens einer Behandlungszone mit Berieselungsdüsen zum Berieseln der Behälter mit einer Prozessflüssigkeit, beispielsweise Wasser, wobei die Behandlungszone eine Abscheideeinheit mit einem Sedimentierungsbereich zum Absetzen von Sediment aus der Prozessflüssigkeit umfasst; mit einem Umlaufkreislauf zum Wiederverwenden der Prozessflüssigkeit; mit Mitteln zum zonenweisen Abziehen des Sediments aus dem Sedimentierungsbereich und zum Zuführen des Sediments an eine zentrale Filtereinheit; wobei die zentrale Filtereinheit wenigstens ein Filtermodul zum Filtern von Feststoffen aus dem zugeführten Sediment umfasst, so dass gefilterte Prozessflüssigkeit erhalten wird; und mit Mitteln zum Rückleiten der gefilterten Prozessflüssigkeit an eine oder mehrere Behandlungszonen.

## Beschreibung

### Gebiet der Erfindung

Die vorliegende Erfindung betrifft ein Temperierungssystem mit Reinigung der Prozessflüssigkeit.

### Hintergrund der Erfindung

Vorrichtungen zur thermischen Behandlung von Produkten, die in Behältern, beispielsweise Flaschen, PET-Behältern oder Dosen abgefüllt sind, sind im Stand der Technik bekannt. Beispielsweise können diese Vorrichtungen Pasteurisationssysteme, Wärme- oder Kühlvorrichtungen umfassen. Im Sinne der vorliegenden Anmeldung kann ein Temperierungssystem ein Pasteurisationssystem, kurz Pasteur, ein Wärmesystem oder ein Kühlsystem umfassen. Häufig treten diese Vorrichtungen auch in Kombination in mehrzonigen Temperierungssystemen auf, um die Produkte zumindest zeitweise auf verschiedene, definierte Temperaturniveaus zu bringen. Der Wärmeaustausch erfolgt im Allgemeinen durch Berieselung mit einer Prozessflüssigkeit, beispielsweise Wasser. Unter dem Begriff Berieselung soll das Berieseln oder Besprühen der Behälter verstanden werden. Das im Prozess verwendete Wasser, das auch als Prozesswasser bezeichnet wird, wird typischerweise mittels Düsen über den Produktstrom verrieselt. Dabei gibt das Prozesswasser aufgrund einer Temperaturdifferenz zum Produkt Wärme an das Produkt ab oder nimmt Wärme auf. Das verwendete Wasser wird in der Regel zumindest teilweise wiederverwendet. Es findet ein Umlaufbetrieb des Prozesswassers statt.

Dabei können auf verschiedene Weisen Verunreinigungen in dem Temperierungssystem auftreten, die die Berieselung und letztlich die Funktion des Temperierungssystem empfindlich beeinträchtigen können. Beispielsweise kann in Teilen des Systems oder in einzelnen seiner Zonen ein Biofilm entstehen. Biofilme bestehen typischerweise aus einer Schleimschicht (einem Film), in der Mikroorganismen, wie zum Beispiel Bakterien, Algen, Pilze etc., eingebettet sein können.

Zum Abscheiden von Partikeln, beispielsweise Glasscherben, Sand und/oder Sinkstoffen aus dem Prozesswasser werden typischerweise Siebbänder eingesetzt. Ein zonenübergreifendes Siebband, wie es im vielen Fällen eingesetzt wird, hat den Nachteil, dass hierzu eine horizontale Bandführung notwendig ist und entsprechend dazu die im Wesentlichen vertikale Wasserführung zu einer zusätzlichen Maschinenhöhe führt. Diese zusätzliche Höhe kann beispielsweise bis zu 400 mm betragen und einen erheblichen Mehraufwand im Bereich der Fördereinrichtungen zum Zu- und Abführen der Behälter bedeuten. Bei einer zonenweisen Abscheidung durch je ein Siebband pro Zone sind die anfallenden Kosten zur Abscheidung unverhältnismäßig hoch. Bei einfacheren Maschinen kommen auch Stecksiebe zum Einsatz.

Beide Arten von Sieben haben den Nachteil, dass sie beispielsweise durch Schwebstoffe, Schleimstoffe und auf dem Wasser schwimmende Stoffe verstopfen können. Die genannten Stoffe legen sich beispielsweise auf die Siebflächen und akkumulieren dort. Dort behindern diese Stoffe zunehmend den Durchsatz durch die Siebe. Die Siebe müssen daher regelmäßig auf Verstopfung kontrolliert und gereinigt werden. Insbesondere bei Verwendung von Stecksieben müssen die Stecksiebe gegebenenfalls herausgezogen und gereinigt werden, so dass Systeme mit Stecksieben einen hohen Arbeits- und/oder Personaleinsatz bedingen. Typischerweise wird auch versucht, Schwebstoffe, Schleimstoffe und auf dem Wasser schwimmende Stoffe durch chemische Behandlung aufzulösen. Gelingt es aber beispielsweise nicht, Schleimstoffe oder ähnliche Stoffe durch chemische Behandlung aufzulösen oder gibt es eine Störung in der chemischen Wasserbehandlung, so setzen sich die Siebflächen der genannten Siebarten umso schneller zu.

Die Maschenweite eines Siebes begrenzt die erzielbare Durchflussmenge. Typischerweise können höchstens 2-3 mm große Partikel mit einem Siebband oder Stecksieb gefiltert werden. Nadelförmige Glasscherben können solche Maschen unter Umständen noch passieren, was dann zu einer Verstopfung der Düsen für die Berieselung führen kann. Ferner sind die Düsen wie auch Pumpenlaufräder häufig aus Kunststoff gefertigt. Durch Scherben oder andere feste Stoffe mit weniger als 2-3 mm Durchmesser können aber Kunststoffdüsen von innen her verschleißen. Jedoch ist ein regelmäßiger Austausch der Düsen ebenso unerwünscht wie ein unkontrolliertes Spritzen aus ausgefransten oder beschädigten Düsen. Verwendet man jedoch Frischwasser zur Innenreinigung, hat man häufig nur kaltes Wasser zur Verfügung. Das zur Reinigung eingesetzte Wasser ist in der Regel verloren, wodurch zusätzliche Wasserverbrauchskosten entstehen. Kaltes Wasser reinigt zudem nicht so gut wie warmes Wasser. Entsprechend wird dann für kaltes Wasser ein höherer Spritzdruck benötigt. Verwendet man das Kreislaufwasser der Spritzungen, sammelt sich der abgelöste Schmutz in den Stecksieben und muss später manuell entfernt werden. Wenn man die Spritzungen laufen lässt, kann man, bedingt durch den erforderlichen Wasserspiegel in der jeweiligen Zone des Systems, Flächen, die unterhalb der Wasserlinie liegen, nur sehr schwer reinigen. Um solche Fläche zu reinigen, ist es häufig notwendig, aufgefangenes Wasser ablaufen zu lassen und dann diese Flächen manuell zu reinigen. Dann wird jedoch Frischwasser mit hohem Druck benötigt, um Biofilme an den unter der Wasserlinie liegenden Wänden ablösen zu können. Somit werden Arbeitszeit, Wasser, und Energie in hohem Maße benötigt, um eine Reinigung dieser Fläche durchführen zu können. Dosiert man Biozid im Tank einer Zone, wird das Biozid mit dem Prozesswasser von der Spritzwasserpumpe angesaugt und über den zu reinigenden Flächen verrieselt. Flächen, die im Sprühschatten der Berieselung stehen oder der Behandlungsraum oberhalb der Spritzrohre weisen regelmäßig starke Verunreinigungen durch Biofilm auf. Nach einer Reinigung, bei der diese Bereiche nur ungenügend erreicht werden, können diese Bereiche zu einer erneuten Verkeimung des Termperierungssystems führen. Erneut müssen diese Bereiche dann unter zum Teil erheblichen Aufwand manuell gereinigt werden. Die oft eingesetzte thermische Desinfektion erfordert erhebliche thermische Energie, insbesondere um das System praktisch komplett zu erhitzen und alle Zonen auf die Desinfektionstemperatur zu bringen. Zusätzlich ist damit ein erheblicher Zeitaufwand außerhalb der Produktion verbunden. Erwärmung und Einwirkung benötigen häufig mehrere Stunden. Typischerweise ist das temperierte Wasser vorher zu entleeren. Man muss also für die Desinfektion praktisch den gesamten Wasserinhalt des Systems austauschen. Auch das kostet zusätzlich Betriebszeit zum Füllen und Entleeren und entsprechende Wasserkosten. Diese Prozedur ist für den Betreiber daher in jeder Hinsicht extrem kostenintensiv.

Angesichts der oben zitierten Probleme ist es Aufgabe der vorliegenden Erfindung, ein Temperierungssystem mit Reinigung der Prozessflüssigkeit vorzusehen, wobei die Hygiene innerhalb des System bei der Behandlung der Behälter verbessert ist und eine geringere Verstopfungsgefahr für das System erreicht wird, so dass das System effizienter wird.

### Beschreibung der Erfindung

Die oben genannte Aufgabe wird durch ein Temperierungssystem mit Reinigung der Prozessflüssigkeit gemäß Anspruch 1 gelöst.

Dabei handelt es sich gemäß der Erfindung um ein Temperierungssystem mit Reinigung der Prozessflüssigkeit, mit einer Zu- und Abführfördereinrichtung für Behälter; mit mindestens einer Behandlungszone mit Berieselungsdüsen zum Berieseln der Behälter mit einer Prozessflüssigkeit, beispielsweise Wasser, wobei die Behandlungszone eine Abscheideeinheit mit einem Sedimentierungsbereich zum Absetzen von Sediment aus der Prozessflüssigkeit umfasst; und mit einem Umlaufkreislauf zum Wiederverwenden der Prozessflüssigkeit; sowie Mittel zum zonenweisen Abziehen des Sediments aus dem Sedimentierungsbereich und zum Zuführen des Sediments an eine zentrale Filtereinheit; wobei die zentrale Filtereinheit wenigstens ein Filtermodul zum Filtern von Feststoffen aus dem zugeführten Sediment umfasst, so dass gefilterte Prozessflüssigkeit erhalten wird; und Mittel zum Rückleiten der gefilterten Prozessflüssigkeit an eine oder mehrere Behandlungszonen.

Die Behälter sind verschlossen. Die Behandlung der Behälter findet durch Berieseln oder Besprühen von außen statt. Die Abscheideeinheit nutzt die Schwerkraftsedimentation insbesondere für kleine Partikel und Sinkstoffe, die in der Prozessflüssigkeit, typischerweise dem Prozesswasser, mitgeführt werden. Diese Partikel und Sinkstoffe sind dichter als die Prozessflüssigkeit und sinken auf den Boden der Abscheideeinheit als Sediment. Die Prozessflüssigkeit wird in einem Umlaufkreislauf geführt und wird typischerweise wieder verwendet. Für eine oder mehrere der Behandlungszonen kann das Sediment mit Hilfe von Mitteln zum Abziehen vom Boden der Behandlungszonen abgezogen werden und einer zentralen Filtereinheit zugeführt werden. Es versteht sich, dass dabei ein Sediment-Prozessflüssigkeit-Gemisch abgezogen wird, welches der Einfachheit halber als Sediment bezeichnet wird. Es werden nicht etwa nur abgesetzte feste Stoffe abzogen. Das Sediment wird der zentralen Filtereinheit zugeführt und dort gefiltert. Dazu umfasst die zentrale Filtereinheit beispielsweise wenigstens ein Filtermodul zum Herausfiltern von Feststoffen aus dem Sediment. Dadurch kann eine gefilterte Prozessflüssigkeit erhalten werden. Die so gefilterte Prozessflüssigkeit kann somit in einem hohen Grade von Feststoffen befreit werden. Die gefilterte Prozessflüssigkeit kann entweder an die Zone zurückgeführt werden, aus der sie entnommen wurde. Es ist ebenso möglich, innerhalb des Systems die gefilterte Prozessflüssigkeit einer oder mehrerer anderer Zonen zurück zu führen.

In dem System können die Mittel zum zonenweisen Abziehen des Sediments aus dem Sedimentierungsbereich ausgebildet sein, eine Wirbelströmung auszubilden, derart dass das Sediment aus dem Sedimentierungsbereich abgezogen werden kann.

In dem System kann die zentrale Filtereinheit ein weiteres Filtermodul zum Filtern von Schwebstoffen umfassen.

Biofilme können sich insbesondere bei bestimmten Umweltbedingungen und bei hinreichender Wärme im Prozesswasser bilden. Die Temperaturen in manchen Behandlungszonen sind geradezu optimal für das Wachstum von Biofilmen. Durch ein derartiges weiteres Filtermodul können Schwebstoffe wie zum Beispiel abgelöster Biofilm und Schleim aus dem Sediment herausgefiltert werden.

In dem System kann die zentrale Filtereinheit ein weiteres Filtermodul umfassen, welches ausgebildet ist, Mikrofiltration und/oder Ultrafiltration und/oder Nanofiltration und/oder Umkehr-Osmosefiltration durchzuführen.

Nährstoffe können mitunter plötzlich und in relativ hoher Konzentration in die Prozessflüssigkeit eingetragen werden, beispielsweise durch lecke oder geplatzte Behälter, so dass sich deren Inhalt mit der Prozessflüssigkeit in den jeweiligen Zonen vermischt. Ein derartiges Filtermodul kann in der Prozessflüssigkeit vorhandene Nährstoffe herausfiltern. Dabei können durch verschiedenen Membrangrößen verschiedene Größen und / oder Arten von Nährstoffen der Prozessflüssigkeit entzogen werden.

In dem System kann die zentrale Filtereinheit ein weiteres Filtermodul zum Bestrahlen der gefilterten Prozessflüssigkeit mit UV-Strahlung umfassen.

Ein weiterer Schritt zur Verbesserung der Hygiene ist eine UV-Bestrahlung der gefilterten Prozessflüssigkeit. Dies geschieht typischerweise, nachdem Feststoffe und/oder Schwebstoffe und/oder Nährstoffe bereits herausgefiltert sind. Durch Bestrahlung mit UV-Licht kann die gefilterte Prozessflüssigkeit desinfiziert werden. Eine Desinfektion ist wünschenswert, weil sie die Neubildung von Biofilm in dem gefilterten Prozesswasser, das den Behandlungszonen rückgeführt wird, reduzieren kann.

Das System kann eine Dosierungseinheit umfassen, die ausgebildet ist, Biozid zu der von der zentralen Filtereinheit gefilterten Prozessflüssigkeit hinzu zu dosieren. Eine weitere, insbesondere ergänzende Möglichkeit zur Desinfektion ist die Zugabe von Biozid in die gefilterte Prozessflüssigkeit. Hierdurch kann die Keimzahl in der gefilterten Prozessflüssigkeit weiter verringert werden. Somit ist die gefilterte Prozessflüssigkeit noch besser desinfiziert und die erneute Keim- und Biofilmbildung wird noch stärker unterdrückt.

In dem System können eine oder mehrere oder alle der Behandlungszonen jeweils ein Innenreinigungsmodul mit einer oder mehreren Düseneinrichtungen umfassen, welche ausgebildet sind, einen oder mehrere Innenbereiche der Behandlungszonen mit gefilterter Prozessflüssigkeit zu reinigen.

Die gefilterte und typischerweise desinfizierte Prozessflüssigkeit kann für die Innenreinigung der Behandlungszonen verwendet werden. Da gefilterte Prozessflüssigkeit, typischerweise Prozesswasser, deutlich reiner und desinfizierter ist, kann sie besonders auch für Reinigungszwecke eingesetzt werden. Ein zusätzlicher Frischwasserverbrauch kann deutlich reduziert oder ganz vermieden werden, denn es wird das gefilterte und desinfizierte Prozesswasser für die Innenreinigung verwendet.

In dem System können wenigstens eine der Düseneinrichtungen ausgebildet sein, die Decke oberhalb der Berieselungsdüsen mit gefilterter Prozessflüssigkeit abzuspritzen.

Oberhalb der Berieselungsdüsen, die für die Behandlung der Behälter in der jeweiligen Behandlungszone eingesetzt werden, entstehen häufig sogenannte Sprüh- oder Berieselungsschatten. Darunter werden Bereiche verstanden, die zwar durch Kondenswasser und/oder Feuchtigkeit benetzt werden, jedoch keine regelmäßige Berieselung erfahren. Hier kann sich besonders gut Biofilm absetzen. Mit einer Düseneinrichtung, die auf diese Bereiche zielt, können diese Bereiche mit dem Prozesswasser gezielt gereinigt werden.

In dem System können in den Behandlungszonen die zu berieselnden Behälter auf mehreren übereinander angeordneten Fördereinrichtungen geführt werden und wenigstens eine der Düseneinrichtungen zwischen zwei übereinander angeordneten Fördereinrichtungen angeordnet sein derart, dass zwischen den Fördereinrichtungen angeordnete Flächen gereinigt werden können.

In dem System kann wenigstens eine der Düseneinrichtungen so angeordnet sein, dass Flächen, die im Betrieb des Systems unter der Wasserlinie liegen, abschwallbar sind.

Bereiche der Zonen, die bei Normalbetrieb unter der Wasserlinie einer Behandlungszone liegen, sind häufig schwierig zu reinigen. Doch auch hier setzen sich Biofilme an den Seitenwänden ab. Wird das gesammelte Wasser in einer Behandlungszone abgelassen, so werden diese Flächen frei. Diese Flächen können dann ebenfalls mit Hilfe von Düseneinrichtungen abgeschwallt werden. Ferner können derartige Düseneinrichtungen so ausgelegt werden, dass sie bereits unter der Wasseroberfläche ein Abschwallen der Seitenwände ermöglichen, wodurch Biofilme und Verschmutzungen der Seitenwände weiter reduziert werden können.

In dem System können die Düseneinrichtungen Rotationsdüsen umfassen, die um 360° drehbar angeordnet sind.

Mittels Rotationsdüsen lassen sich besonders gut alle umliegenden Bereiche abspritzen.

Das System kann ferner eine Steuereinheit umfassen, die ausgebildet ist, die Mittel zum zonenweisen Abziehen des Sediments aus dem Sedimentierungsbereich und zum Zuführen des Sediments an die zentrale Filtereinheit zu steuern.

In dem System kann die Steuereinheit ausgebildet sein, die Temperatur der Prozessflüssigkeit der Behandlungszonen zu messen, und kann ausgebildet sein, gefilterte Prozessflüssigkeit aus wenigstens einer Behandlungszone mit einer höheren Temperatur der Prozessflüssigkeit zur Innenreinigung einer Behandlungszone mit kälterer Prozessflüssigkeit zu verwenden.

Die Steuereinheit kann Ventile steuern und somit das Zuleiten oder Abfließen von Prozessflüssigkeit in die Behandlungszonen steuern. Ebenso kann die Steuereinheit mit Temperatursensoren kommunizieren, die die Temperatur der Prozessflüssigkeit beziehungsweise die Innentemperatur einer Behandlungszone messen können. Somit kann die Steuereinheit regulieren, ob und gegebenenfalls aus welcher Behandlungszone wärmere Prozessflüssigkeit abgezogen, gefiltert und desinfiziert wird, um dann in einer kälteren Behandlungszone wieder verwendet zu werden, insbesondere zur Innenreinigung.

In dem System kann die Abscheideeinheit in der Behandlungszone ferner eine Pumpe und einen unter der Flüssigkeitsoberfläche angeordneten Lamellenschrägklärer mit mehreren parallelen, schräg angeordneten Lamellen umfassen, wobei die Pumpe die Prozessflüssigkeit entlang der Lamellen pumpt.

In dem System kann die Pumpe die Prozessflüssigkeit über den tiefsten Punkt einer Behandlungszone pumpen, so dass sich Sediment absetzen kann.

In den Abscheideeinheiten der Behandlungszonen kann bei allen oder zumindest bei einigen der Abscheideeinheiten eine Filtereinheit in Form eines Lamellenschrägklärers vorgesehen sein. Es handelt sich also um eine zusätzliche, zonenbezogene Filtereinheit. Die Prozessflüssigkeit wird beispielsweise mittels einer Pumpe durch die Abscheideeinheit gepumpt. Die Lamellen können dabei eine große Sedimentationsfläche in kompakter Form bereit stellen, an denen die Prozessflüssigkeit vorbei fließt. An den Lamellen, also an den Sedimentationsflächen können die Partikel sedimentieren und dieses Sediment kann dann durch die Schwerkraft in der Flüssigkeit nach unten auf den Boden der Abscheideeinheit sinken. Es versteht sich, dass die Pumpleistung so gewählt werden kann, dass die absinkenden Partikel nicht durch eine Strömung mitgerissen werden. Die Lamellen können vollständig benetzt sein, also in der Flüssigkeit eingetaucht vorgesehen sein. Dadurch kann die Verschmutzung der Oberflächen der Lamellen deutlich reduziert werden und die Lamellen bleiben praktisch sauber von Rückständen. Rückstände können somit auch nicht auf den Lamellen antrocknen. Aufgrund ihrer Oberflächeneigenschaften können die Lamellen als zusätzliche Sedimentationsflächen wirken. Die Porosität der Oberflächen der Lamellen kann dabei möglichst gering gehalten werden. Hierdurch kann auch das Festsetzen von organischen Schwebstoffen wie etwa Schleim an den Lamellen an den Lamellen deutlich reduziert oder gar vermieden werden. Selbst wenn sich derartige Stoffe in geringem Umfang an den Oberflächen der Lamellen festsetzen würden, würden sie praktisch keine der Sedimentationseigenschaften der Lamellen stören. Die Lamellen können also das Abscheiden von Sediment, das sich dann am Boden der Abscheideeinheit sammeln kann, unterstützen. Somit kann die Effizienz des Systems weiter erhöht werden.

Das Temperierungssystem kann ein Pasteurisationssystem, ein Wärmesystem oder ein Kühlsystem sein.

Es gilt also: Die hier beschriebene Erfindung ermöglicht eine Filtration der Prozessflüssigkeit eines Temperierungssystems und eine Wiederverwenden der gefilterten Prozessflüssigkeit. Da das Sediment abgezogen wird, können der Prozessflüssigkeit mittels der zentralen Filtereinheit Sediment und Schwebstoffe und/oder gelöste Nährstoffe entzogen werden und somit die Prozessflüssigkeit sehr sauber gehalten werden. Dadurch erhöht sich die Standzeit des Systems. Auch nadelförmige Partikel, Scherben etc., die herkömmlich durch ein Stecksieb oder ein Filterband des Temperierungssystems hindurch gehen könnten, können durch die Sedimentation erfasst werden und mittels der zentralen Filtereinheit heraus gefiltert werden. Hierdurch können Verstopfungen oder Beschädigungen von Berieselungsdüsen weitestgehend vermieden werden. Insbesondere ermöglicht die Erfindung eine Innenreinigung von Behandlungszonen des Systems praktisch ohne zusätzlichen Frischwasserverbrauch. Die Innereinigung kann beispielsweise im laufenden Betrieb erfolgen. Eine unzulässige Vermischung der Temperaturen kann durch die Steuerung der Kreislaufführung praktisch ausgeschlossen werden. Der von den Flächen bei der Reinigung abgetragene Schmutz kann über den Kreislauf der Prozessflüssigkeit mittels der zentralen Filtereinrichtung herausgefiltert und schließlich entfernt werden. Durch Hinzugeben von Biozid zur gefilterten Prozessflüssigkeit, kann über die Reinigungsdüsen Biozid in den jeweiligen Behandlungszonen dorthin gebracht werden, wo es normalerweise nicht hinkommt. Die Reinigung mit UV-Strahlen kann ebenfalls biozid wirken, da durch UV-Strahlen freie Radikale erzeugt werden können, die wiederum biozid wirken können. Durch Zuführung dieser freien Radikale bei der Innenreinigung können diese freien Radikale direkt den zu reinigenden Flächen zugeführt werden. Hierdurch kann der Verbrauch an Chemikalien zur Desinfektion reduziert werden. Für die Reinigung von Flächen unterhalb der Wasserlinie kann nach Entleeren der Behandlungszonen heißes Wasser aus den jeweiligen Temperierungszonen verwendet werden. Somit kann thermische Energie gespart werden. Herkömmlicherweise müsste das System zonenweise, abschnittsweise oder sogar komplett erhitzt werden, um sie thermisch zu desinfizieren. Somit können Betriebskosten aus Wasser, Strom und Arbeitszeit, die durch den erzwungenen Betriebsstillstand zur thermischen Desinfektion und zur manuellen Reinigung des Systems anfallen, reduziert werden. Wartungsintervalle können verlängert werden oder in manchen Fällen praktisch völlig eingespart werden.

Bei einem Pasteurisationssystem werden Produkte beispielsweise aufgeheizt um eine Pasteurisierung zu erreichen und beispielsweise anschließend mit dem System auch wieder abgekühlt und dann ausgegeben. Dazwischen kann eine Temperatur auch gehalten werden, um den Pasteurisierungseffekt zu verbessern. Das Aufheizen und das Abkühlen (und auch das Halten) kann in jeweils mehreren Temperaturzonen erfolgen. Dabei können zum Beispiel die Aufwärm- und die Abkühlzonen miteinander regenerativ verbunden sein, was bedeutet, das Wasser, dass sich beim Abkühlen der Produkte selber aufgewärmt hat, zum Aufwärmen von einlaufenden (kälteren) Produkten in einer der Aufwärmzonen verwendet wird.

Bei einem Wärmesystem werden Produkte lediglich aufgewärmt und dann ausgegeben (also nicht abgekühlt). Dies kann zum Beispiel eingesetzt werden, um mit kaltem Inhalt gefüllte Produkte aufzuwärmen, um eine Kondensationswasserbildung oder -erhaltung auf den Produkten zu vermeiden. Auch hier können mehrere Aufwärmzonen vorgesehen sein. Eine regenerative Verbindung dieser Aufwärmzonen ist hierbei aber beispielsweise nicht vorgesehen.

Bei einem Kühlsystem werden Produkte lediglich abgekühlt (also nicht erst erwärmt) und dann ausgegeben. Dies kann z.B. dazu dienen heißabgefüllte Produkte möglichst schnell kühl zu bekommen, beispielsweise um die Wärmeeinwirkung auf das Produkt oder auf den Behälter möglichst kurz bzw. gering zu halten. Auch hier können mehrere Abkühlzonen vorgesehen sein. Eine regenerative Verbindung dieser Abkühlzonen ist hierbei aber beispielsweise nicht vorgesehen.

Im Folgenden werden Ausführungsformen der Erfindung unter Bezugnahme auf die Zeichnungen beschrieben. Die beschriebenen Ausführungsformen sind in jeder Hinsicht lediglich als illustrativ und nicht als einschränkend anzusehen und verschiedene Kombinationen der angeführten Merkmale sind in der Erfindung eingeschlossen.

### Kurzbeschreibung der Figuren

Figur 1 zeigt eine Prinzipskizze eines Temperierungssystems gemäß der vorliegenden Erfindung.
Figur 2 zeigt eine Weiterbildung des in Figur 1 skizzierten Temperierungssystems.
Figur 3 zeigt eine weitere Weiterbildung des in den Figuren 1 und 2 gezeigten Temperierungssystems.
Figur.4 zeigt ein Temperierungssystem mit mehreren Behandlungszonen, das dem Temperierungssystem aus Figur 3 entspricht.
Figur 5 zeigt einen Ausschnitt aus dem Temperierungssystem aus Figur 4, bei dem zusätzlich ein Lamellenschrägklärer vorgesehen ist.

### Detaillierte Beschreibung

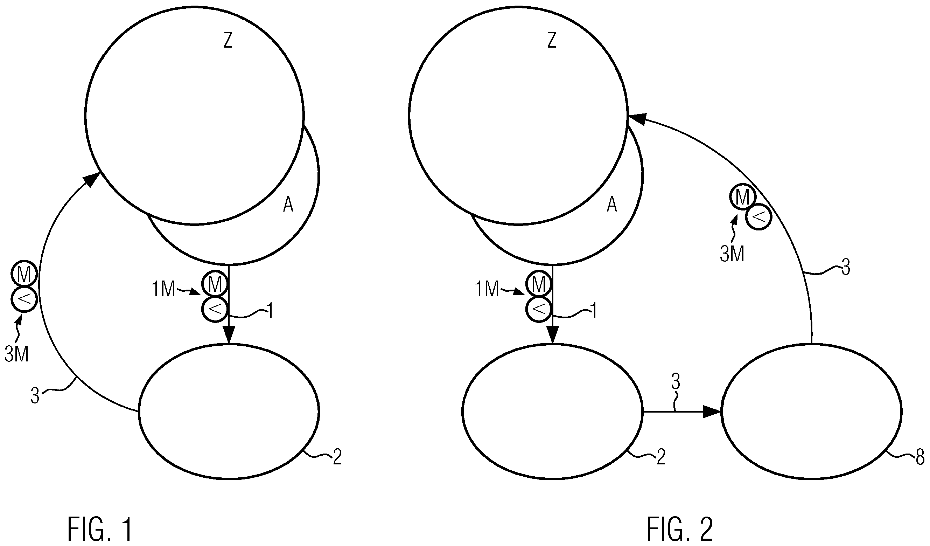

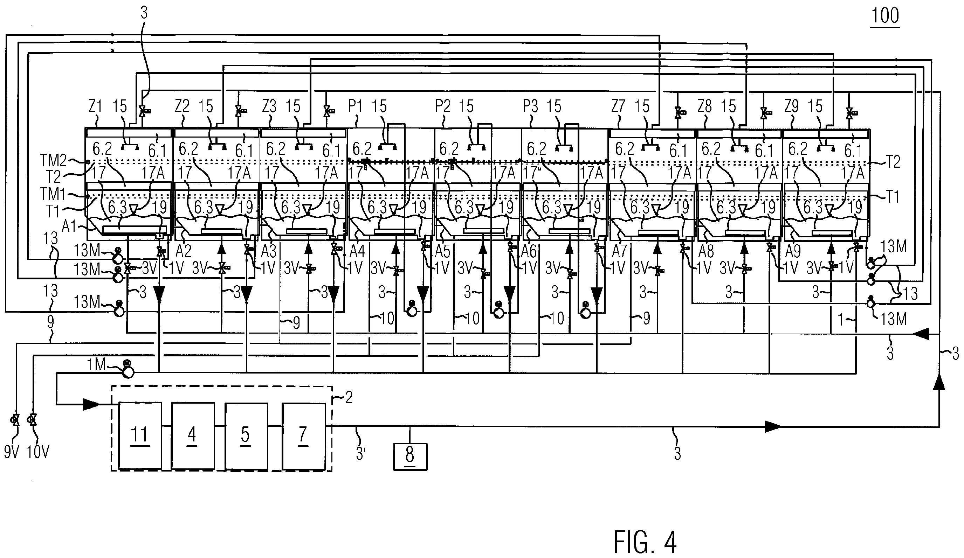

Die Figur 1 zeigt eine Prinzipskizze eines Temperierungssystems gemäß der vorliegenden Erfindung. Ein Bereich mit wenigstens einer Behandlungszone Z wird zum Behandeln von in Behältern abgefüllten Flüssigkeiten eingesetzt. Die Behälter sind verschlossen. Typischerweise werden die Behälter mit Prozessflüssigkeit, insbesondere Prozesswasser berieselt. Details sind in Figur 4 dargestellt. Die Behälter sind in Figur 1 nicht explizit gezeigt. Bei der Behandlung der Behälter können sich beim Berieseln der Behälter Schmutz oder Partikel von den Behältern ablösen. In dem Temperierungssystem der Figur 1 gibt es eine Abscheideeinheit A zum Abscheiden oder Absetzen von Sediment aus der Prozessflüssigkeit. Typischerweise umfasst die Abscheideeinheit A einen Siebkasten oder Abscheidekasten, siehe Figur 4. Das Temperierungssystem umfasst Mittel 1 zum Abziehen des Sediments, hier etwa eine Rohrleitung 1 mit einer Pumpe 1M. Mit Hilfe der Pumpe 1M wird das Sediment aus dem Siebkasten abgezogen und in eine zentrale Filtereinheit 2 geführt. Die zentrale Filtereinheit 2 umfasst zumindest ein Filtermodul zur Filtration von Feststoffen, siehe Figur 4. Beispielsweise kann das Filtermodul der zentralen Filtereinheit einen Spaltfilter mit einer definierten Spaltgröße umfassen. Beispielsweise kann die Spaltgröße 40 - 60 µm betragen. Es sind aber auch andere Spaltgrößen möglich. Teilchen, die in diesem Filtermodul zurück gehalten werden, können durch Umkehrspülung aus dem Filtermodul gelöst werden, beispielsweise mit Hilfe einer pneumatischen Umkehrspülung. Somit kann das Filtermodul der Filtereinheit 2 mittels pneumatischen Umkehrstoßes gereinigt und mehrfach verwendet werden. Die zentrale Filtereinheit 2 der Figur 1 kann weiter Filtermodule enthalten, die anhand von Figur 4 erläutert werden. Insbesondere kann die zentrale Filtereinheit ein UV-Modul enthalten zum Bestrahlen der gefilterten Prozessflüssigkeit, wodurch Bakterien und Pilze abgetötet werden können.

Die Figur 1 zeigt ferner Mittel 3, beispielsweise eine Rohrleitung 3 mit einer Pumpe 3M, zum Rückleiten des gefilterten Prozesswasser an die Behandlungszone Z. Somit kann das gefilterte Prozesswasser in gefilterter, also gereinigter Weise, erneut in der Behandlungszone Z verwendet werden. Es besteht also ein im Wesentlichen geschlossener Kreislauf für das Prozesswasser. Dabei soll "geschlossener Kreislauf" so verstanden werden, dass keine großen Frischwassermengen nachgeliefert werden brauchen.

Die Figur 2 zeigt eine Weiterbildung der Ausführungsform, die in der Figur 1 skizziert ist. Dabei sind gleiche Elemente mit gleichen Bezugszeichen bezeichnet. In Figur 2 ist für die Rückführung der gefilterten Prozessflüssigkeit zwischen der Filtereinheit 2 und der Behandlungszone Z eine Desinfektionseinheit 8 gezeigt. Die Desinfektionseinheit 8 ist beispielsweise ausgebildet, in dosierter Weise dem Prozesswasser Biozid zuzugeben. Dadurch kann eine Desinfektion oder höher Sterilisation des Prozesswassers erzielt werden. Es versteht sich, dass die Zugabe von Biozid von einer Steuereinheit (nicht gezeigt) gesteuert werden kann. Das gefilterte und dann desinfizierte Prozesswasser wird über eine Rohrleitung 3 mit Pumpe 3M wieder an die Behandlungszone Z zurück geleitet, wo es erneut verwendet werden kann.

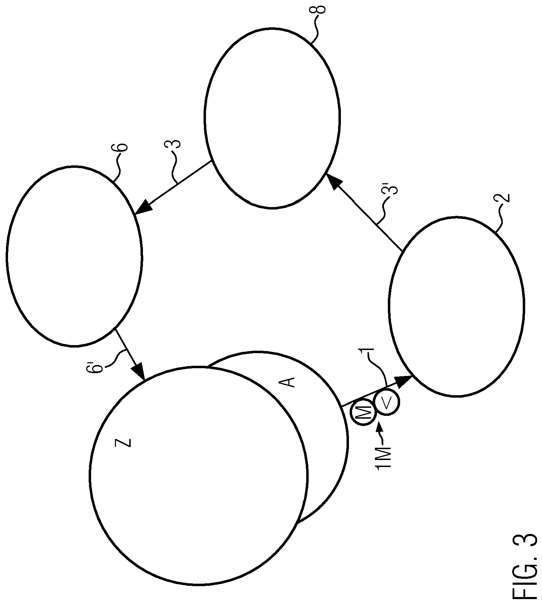

Die Figur 3 zeigt eine weitere Weiterbildung der Ausführungsformen aus den Figuren 1 und 2. Dabei sind erneut gleiche Elemente mit gleichen Bezugszeichen bezeichnet. Die Figur 3 zeigt sämtliche Elemente der Figur 2. Zusätzlich ist in der Figur 3 noch ein Bereich 6 bezeichnet, welcher die Innenreinigung des Temperierungssystems bezeichnet. Der Bereich 6 umfasst also Vorrichtungen, die die Innenreinigung der Behandlungszone Z des Temperierungssystems betreffen. Insbesondere kann das gefilterte und gereinigte Prozesswasser für die Innenreinigung im Bereich 6 eingesetzt werden. Dadurch wird erneut praktisch kein zusätzliches Frischwasser für die Innenreinigung benötigt, sondern es kann das gefilterte und desinfizierte Prozesswasser verwendet werden. Es versteht sich, dass zusätzlich zu den Elementen Filtereinheit 2, Desinfektionseinheit 8 und Bereich 6 noch ein weiterer Filterzug parallel aufgebaut sein kann (nicht gezeigt), der nur einige der gezeigten Elemente umfasst. Insbesondere kann ein Teil des Prozesswassers auch direkt zur erneuten Berieselung verwendet werden.

Die Figur 4 zeigt eine weitere Weiterbildung der Ausführungsform aus Figur 3.

In Figur 4 ist ein Temperierungssystem 100 dargestellt. Rein beispielhaft ist das Temperierungssystem 100 aus drei Abschnitten aufgebaut. Die Abschnitte umfassen: einen ersten Abschnitt zur Erwärmung / Anwärmung der zu behandelnden Behälter durch Berieseln mit Prozesswasser; einen zweiten Abschnitt, in dem die zu behandelnden Behälter mit warmem Prozesswasser berieselt werden und einem dritten Abschnitt, in dem die zu behandelnden Behälter durch Berieseln mit Prozesswasser abgekühlt werden. Rein beispielhaft umfasst in Figur 4 jeder der Abschnitte 3 Zonen. Es versteht, sich, dass jeder der Abschnitte auch eine andere Zahl von Zonen umfassen könnte. Auch können die verschiedenen Abschnitte eine verschiedene Anzahl von Behandlungszonen umfassen. Zu behandelnde Behälter werden typischerweise auf mindestens einer Fördereinrichtung durch die Zonen geführt. In Figur 4 sind beispielhaft zwei übereinander angeordnete Fördereinrichtungen oder Förderbänder T1 und T2 gezeigt. Diese Förderbänder werden in Figur 4 geeignet angetrieben. Beispielhaft sind Motoren TM1 und TM2 zum Antreiben der Förderbänder T1 respektive T2 gezeigt. Auf den Förderbänder T1 und T2 können somit in zwei Etagen oder zwei Decks zu behandelnde Behälter durch die Behandlungszonen transportiert werden.

Der erste Abschnitt umfasst die Zonen Z1, Z3, Z3. In diesen Zonen findet eine Anwärmung der zu behandelnden Behälter durch Berieseln statt. Der zweite Abschnitt umfasst die Zonen P1, P2, P3. In diesen Zonen wird typischerweise mit hinreichend warmen Prozesswasser die Temperierung durchgeführt. Diese Zonen P1, P2, P3 können auch als Temperierungszonen bezeichnet werden. Auf die Zonen P1, P2 und P3 folgen im dritten Abschnitt die Zonen Z7, Z8 und Z9. In diesen letzten drei gezeigten Zonen findet die Abkühlung der zuvor mit warmem Wasser behandelten Behälter statt. In den Zonen Z7, Z8 und Z9 werden die Behälter mit kühlerem Wasser zur Abkühlung berieselt. Die Zonen P1, P2, P3 schließen typischerweise unmittelbar an die Zonen Z1, Z2, Z3 an. Das heißt, die Förderbänder, hier T1 und T2, führen in Figur 4 die zu behandelnden Behälter von den Zonen Z1, Z2, Z3 in die Zonen P1, P2 und P3 und dann in die Zonen Z7, Z8 und Z9. Das Prozesswasser wir aus Berieselungsvorrichtungen 15 auf die Behälter gesprüht. Die Berieselungsanlagen 15 sind typischerweise oberhalb der zu behandelnden Behälter vorgesehen und berieseln die Behälter im Wesentlichen von oben oder schräg von der Seite.

Die in Figur 4 gezeigten Zonen Z1, Z2, Z3, P1, P2, P3, Z7, Z8, Z9 haben jeweils Auffangzonen mit Abscheideeinheiten A1, A2, A3, A4, A5, A6, A7, A8, und A9. Diese Abscheideeinheiten sind wannenartig ausgebildet. In diesen Abscheideeinheiten A1, A2, A3, A4, A5, A6, A7, A8, und A9 sammelt sich jeweils das in der entsprechenden Behandlungszone Z1, Z2, Z3, P1, P2, P3, Z7, Z8, und Z9 Prozesswasser 17 nach Gebrauch. Durch den Kontakt mit den zu behandelnden Behältern entstehen Einträge von Partikeln, beispielsweise Glasscherben, Sand und/oder Sinkstoffen in das Prozesswasser 17. Gleichzeitig können organische Schwebstoffe an den Behältern vorhanden sein, die sich teilweise ablösen und dann in das gebrauchte Prozesswasser 17 gelangen. Aufgrund des feuchten und warmen Milieus in den Behandlungszonen, besonders in den warmen Behandlungszonen, können sich Biofilme an Seitenwänden der jeweiligen Behandlungszonen bilden. Teile dieser Biofilme können sich ablösen und in das benutzte Prozesswasser 17 gelangen, das in den Abscheideeinheiten A1, A2, A3, A4, A5, A6, A7, A8, und A9 der jeweiligen Behandlungszonen Z1, Z2, Z3, P1, P2, P3, Z7, Z8, Z9 gesammelt wird. Einträge aus Partikeln, Sand und/oder Sinkstoffen, die auch organische Sinkstoffe umfassen können, sinken in den Abscheideeinheiten der jeweiligen Behandlungszone nach unten. Die Abscheideeinheiten A1, A2, A3, A4, A5, A6, A7, A8, und A9 umfassen jeweils an ihrem tiefsten Punkt Siebkästen oder Auffangbehälter 19 zur Aufnahme der Einträge, d.h. zur Aufnahme des Sediments. Jeder der Abscheidebehälter 19 in den unterschiedlichen Behandlungszonen Z1, Z2, Z3, P1, P2, P3, Z7, Z8, Z9 kann in unterschiedlichem Maße mit Sediment gefüllt sein. Das Sediment kann aus den Abscheidebehälter 19 mit Hilfe von Mitteln zum Abziehen des Sediments abgezogen werden. Die Mittel zum Abziehen des Sediments können beispielsweise Pumpen und Ventile umfassen. In Figur 4 sind zwecks Sedimentabzugs Ventile 1V und mindestens eine Pumpe 1M zum Abziehen des Sediments aus den jeweiligen Abscheideeinheiten beziehungsweise deren Abscheidebehälter 19 gezeigt. Die Ventile 1V und die mindestens eine Pumpe 1M können von einer Steuereinheit (nicht gezeigt) individuell steuerbar sein. Somit kann jeweils eine oder gegebenenfalls auch mehrere Abscheidebehälter für den Sedimentabzug geöffnet werden und zwecks Filterung des Sediments weitergeleitet werden. Das Sediment kann durch eine Pumpe 1M abgezogen werden, welche geeignet ist, eine Wirbelströmung auszubilden, so dass das Sediment aus dem Sedimentierungsbereich abgezogen werden kann. Dabei versteht sich, dass ein Gemisch aus Prozesswasser 17 und festen Teilchen sowie in dem Prozesswasser 17 gelösten Teilchen abgezogen werden kann.

Das abgezogenen Prozesswasser und Sediment werden über Leitungen 1 zu einer zentralen Filtereinheit 2 geleitet. Die zentrale Filtereinheit 2 ähnelt der zentralen Filtereinheit 2 in den Figuren 1 - 3. Die zentrale Filtereinheit 2 umfasst typischerweise ein oder mehrere Filtermodule. Ein erstes Filtermodul 11 der zentralen Filtereinheit 2 ist typischerweise ein Filtermodul 11 zur Filtration von Feststoffen. Dieses Filtermodul 11 kann, wie bereits anhand von Figur 1 beschrieben, einen Spaltfilter mit einer definierten Spaltgröße umfassen. Beispielsweise kann die Spaltgröße 40 - 60 µm betragen. Es sind jedoch auch andere Spaltgrößen möglich. Somit können Partikel mit mittleren Partikeldurchmessern, die größer als die Spaltgrößen sind, aus dem Sediment herausgefiltert werden. Somit wird das aus der jeweiligen Behandlungszone abgezogene Prozesswasser im Hinblick auf feste Stoffe, also Partikel, gefiltert.

In der zentralen Filtereinheit in Figur 4 kann dem ersten Filtermodul 11 ein zweites Filtermodul 4 nachgeordnet sein. Das zweite Filtermodul 4 kann insbesondere auf die Filtration von Schwebstoffen und Teilen von Biofilmen abstellen. Schwebstoffe, Schleimstoffe und auf beziehungsweise im Wasser schwimmende, in der Regel organische Stoffe können mit Hilfe dieses Filtermoduls aus dem Prozesswasser weitestgehend entfernt werden.

In der zentralen Filtereinheit in Figur 4 kann dem ersten oder dem zweiten Filtermodul ein drittes Filtermodul 5 folgen. Das dritte Filtermodul 5 kann insbesondere ausgebildet sein, Nährstoffe aus dem Prozesswasser herauszufiltern. In dem System kann die zentrale Filtereinheit ein weiteres Filtermodul umfassen, welches ausgebildet ist, Mikrofiltration und/oder Ultrafiltration und/oder Nanofiltration und/oder Umkehr-Osmosefiltration durchzuführen. Nährstoffe können plötzlich und in relativ hoher Konzentration in die Prozessflüssigkeit eingetragen werden, beispielsweise durch lecke oder geplatzte Behälter. Der Inhalt lecker oder geborstener Behälter kann sich mit dem gebrauchten Prozesswasser 17 in den Abscheideeinheiten der Behandlungszonen vermischen. Dabei können Membranfilter eingesetzt werden. Verschiedenen Membrangrößen können dem Prozesswasser verschiedene Arten von Nährstoffen entziehen. Zum Beispiel können in dem Filtermodul 5 ein oder mehrere Untermodule zur Mikrofiltration und/oder Ultrafiltration und/oder Nanofiltration und/oder Umkehr-Osmosefiltration eingesetzt werden. Dabei umfasst Mikrofiltration eine Größe der abtrennbaren Stoffe bis etwa größer gleich 0,1 µm bei Druckdifferenzen von 0,1 - 2 bar. Ultrafiltration umfasst eine Größe der abtrennbaren Stoffe bis etwa eine Größenordnung geringer (also etwa 0,01 µm) als bei Mikrofiltration bei Druckdifferenzen von 0,1 - 5 bar. Nanofiltration umfasst eine weitere Größenordnung geringer als Ultrafiltration (also etwa bis 0,001 µm) bei Druckdifferenzen von 3 - 30 bar. Umkehrosmosefiltration ist noch eine Größenordnung geringer als Nanofiltration (also bis etwa 0,0001 µm) bei Druckdifferenzen von 10 - 100 bar. Somit können Nährstoffe dem Prozesswasser entzogen werden. Da die im Prozesswasser vorhandenen Nährstoffe Bakterien, Biofilme und andere organische Formen im Prozesswasser nähren können, kann durch das Herausfiltern der Nährstoffe diesen unerwünschten Formen Nahrung entzogen werden.

Die zentrale Filtereinheit 2 kann ein viertes Filtermodul 7 umfassen, das eine UV-Bestrahlungseinrichtung zum Bestrahlen des Prozesswassers umfasst. Typischerweise kann das Filtermodul 7 nach den Filtermodulen 11, 4, und 5 vorgesehen sein. Es kann auch als eine separate Einheit den übrigen drei Filtermodulen nachgeschaltet sein. Durch die UV-Bestrahlung kann eine keimtötende Wirkung erzielt werden. Somit kann die UV-Bestrahlung das vorgefilterte Prozesswasser desinfizieren. Dadurch können bereits in der zentralen Filtereinheit Keime abgetötet werden. Durch UV-Strahlen können freie Radikale erzeugt werden, die biozid wirken können. Sofern zur Desinfektion des Prozesswassers auch Chemikalien oder biozide Stoffe zugegeben werden, siehe unten, kann die Menge die zuzugebenden Stoffe durch den Einsatz von UV-Bestrahlung effizient reduziert werden. Es versteht sich, dass die UV-Bestrahlungsvorrichtung, also das vierte Filtermodul 7 auch derart in die zentrale Filtereinheit integriert sein kann (in Figur 4 nicht gezeigt), dass während der Filtration des Prozesswasser mit einem oder mehreren oder allen der übrigen Filtermodul 11, 4, und 5, eine UV-Bestrahlung im Wesentlichen zeitgleich erfolgen kann. Es versteht sich, dass dabei die UV-Bestrahlung mittels einer Steuereinheit gezielt ein- oder ausgeschaltet werden kann. Nachdem das aus der jeweiligen Abscheideeinheit abgezogene Sediment mit Prozesswasser die zentrale Filtereinheit 2 mit ihren Modulen 11, 4, 5 und 7 durchlaufen hat, wird gefiltertes Prozesswasser von der zentralen Filtereinheit 2 ausgegeben. Dieses kann durch Pumpen (nicht gezeigt) zur weiteren Verwendung weitergeleitet werden.

In dem Temperierungssystem 100 in Figur 4 ist nach der zentralen Filtereinheit 2 eine Dosierungseinheit 8 gezeigt. Diese ist der zentralen Filtereinheit 2 nachgeschaltet. Mittels der Dosierungseinheit 8 kann dem gefilterten Wasser ein Biozid in dosierter Form zugegeben werden. Dadurch kann zum einen das gefilterte Wasser noch weiter desinfiziert werden. Zum anderen kann das gefilterte Prozesswasser auch als Träger für das Biozid wirken. Das Biozid kann bei der Wiederverwendung des Prozesswassers dorthin gebracht werden, wo Flächen innerhalb der Behandlungszonen mit dem gefilterten Prozesswasser abgespritzt werden sollen, beispielsweise im Hinblick auf eine Innenreinigung, wie nachfolgend beschrieben, oder ein Durchspülen von Leitungen. Dabei kann das gezielte Dosieren des Biozids durch die Dosierungseinheit 8 mittels einer Steuereinheit gesteuert werden.

In dem Temperierungssystem 100 in Figur 4 wird das gefilterte Prozesswasser über Leitungen 3 den Behandlungszonen Z1, Z2, Z3, P1, P2, P3, Z7, Z8, und Z9 zurück geleitet. Dabei kann die Rückleitung in die Behandlungszonen Z1, Z2, Z3, P1, P2, P3, Z7, Z8, und Z9 jeweils über Ventile 3V gesteuert werden.

In den Behandlungszonen Z1, Z2, Z3, P1, P2, P3, Z7, Z8, und Z9 sind Reinigungseinheiten zur Innenreinigung der jeweiligen Zonen vorgesehen. Im Beispiel in Figur 4 sind in den Zonen Z1, Z2, Z3, Z7, Z8 und Z9 jeweils Reinigungseinheiten 6.1 und 6.2 und 6.3 vorhanden. In den Zonen P1, P2 und P3 sind nur Reinigungseinheiten 6.2 und 6.3 vorhanden. Es versteht sich, dass auch eine andere Anzahl von Reinigungseinheiten möglich sein kann. Die Reinigungseinheiten 6.1 umfassen Düsenvorrichtung zum Abspritzen der Decke und oder der deckennahen Seitenwände der jeweiligen Behandlungszonen Z1, Z2, Z3, Z7, Z8 und Z9. Die Reinigungseinheiten 6.1 sind typischerweise oberhalb der Berieselungsdüsen 15 vorgesehen.

Das gefilterte Prozesswasser, das typischerweise auch Biozid enthält, kann somit Deckenbereiche der jeweiligen Behandlungszone erreichen, die im normalen Berieselungsbetrieb größtenteils abgeschattet sind, d.h. in diese Bereiche gelangt zwar Feuchtigkeit und Wärme, jedoch kaum Prozesswasser aus den Berieselungsdüsen 15.

Als Düsenvorrichtung für die Reinigungsspritzung können beispielsweise rotierende Düsen verwendet werden, die um 360° rotierbar sind. Somit können praktisch alle Bereiche oberhalb der Berieselungsdüsen 15 gereinigt werden. Das bei der Reinigung verwendete Prozesswasser sowie die damit abgelösten oder darin teilweise gelösten Schmutzpartikel oder Biofilmanteile gelangen erneut in die Abscheideeinheiten A1, A2, A3, A4, A5, A6, A7, A8, und A9 und können wiederum durch Abziehen der zentralen Filtereinheit 2 zugeleitet werden.

Die Reinigungseinheiten 6.2 sind zwischen den Fördereinrichtungen T1 und T2 vorgesehen und können dort die Seitenwände oder die Unterseiten der Fördereinrichtungen T1 und T2 abspritzen. Somit können Seitenbereiche oder Unterseiten, die im Berieselungsbetrieb kaum mit Prozesswasser berieselt werden können, mit Hilfe der Reinigungseinheiten 6.2 abgespritzt und somit gereinigt werden. Die Reinigungseinheiten 6.2 können gleichzeitig oder gegebenenfalls separat von den Reinigungseinheiten 6.1 mit gefiltertem Prozesswasser versorgt werden. Die Reinigungseinheit 6.2. können ähnlich wie die Reinigungseinheiten 6.1 rotierende Düsen verwenden, die um 360° rotierbar sind, so dass zwischen den beiden Fördereinrichtungen T1 und T2 praktische sämtliche Bereiche mittels der rotierenden Düsen abgespritzt werden können. Es versteht sich, dass in einer Einheit, in der nur eine Fördereinrichtung vorhanden ist (hier nicht gezeigt), eine Reinigungseinheit wie die Reinigungseinheit 6.2 typischerweise unterhalb der Fördereinrichtung vorgesehen sein kann.

In den Behandlungszonen Z1, Z2, Z3, P1, P2, P3, Z7, Z8, und Z9 sind die Reinigungseinheiten 6.3 im Bereich der Abscheideeinheiten A1, A2, A3, A4, A5, A6, A7, A8 und A9 vorgesehen. Das Besondere an den Reinigungseinheiten 6.3 ist, dass sie in einem Bereich vorgesehen sind, der im normalen Berieselungsbetrieb unterhalb der Wasserlinie 17A der Prozessflüssigkeit 17 in der jeweiligen Behandlungszone liegt. Das Prozesswasser 17 aus einer Behandlungszone kann jedoch abgelassen werden. Über die Leitung 9 kann in Figur 4 das gesammelte, gebrauchte Prozesswasser 17 aus einer oder mehreren oder allen der Abscheideeinheiten A1, A2, A3, A7, A8 und A9 abgelassen werden, die im Wesentlichen kaltes gebrauchtes Prozesswasser 17 auffangen. Analog kann über die Leitung 10 das gesammelte, gebrauchte Prozesswasser 17 aus einer oder mehreren oder allen der Abscheideeinheiten A4, A5, und A6 abgelassen werden, die im Wesentlichen warmes Prozesswasser 17 auffangen. Eine dann geleerte Abscheideeinheit kann dann ebenfalls mittels einer Düseneinheit 6.3 abgeschwallt werden, so dass Flächen in der jeweiligen Behandlungszone gereinigt werden können, die normalerweise unter der Wasserlinie liegen. Somit kann die Hygiene in der jeweiligen Behandlungszone weiter verbessert werden.

Mittels einer zentralen Steuereinheit (nicht gezeigt) kann die Innenreinigung der jeweiligen Behandlungszonen in automatisierter Form gesteuert werden. Dabei kann die Innenreinigung praktisch im laufenden Betrieb des Temperierungssystems automatisch erfolgen, sofern das Folgende berücksichtigt wird. Bei laufendem Betrieb des Systems kann "eine Lücke" gefahren werden. Damit ist gemeint, dass auf den Förderbändern für eine gewisse Zeit, die bei gleichbleibender Fördergeschwindigkeit einer gewissen räumlichen Breite entspricht, keine Flaschen oder Behälter auf dem Förderband stehen. Beispielsweise kann diese räumliche Breite die Breite von ein bis zwei Breiten einer der Behandlungszonen umfassen. Eine solche Lücke kann auch entstehen, wenn ein sogenannter Produktwechsel erfolgt. Das bedeutet, es wird von der Berieselung einer Sorte von Behältern auf eine andere Sorte von Behältern umgestellt. Die Lücke wird dahingehend ausgenutzt, dass in der Behandlungszone, in der gerade keine Behälter besprüht werden müssen, was der Lücke entspricht, die Innenreinigung der Behandlungszone durchgeführt werden kann. Eine Steuereinheit kann die Steuerung der Innenreinigung übernehmen. Das bedeutet insbesondere das Umschalten auf Innenreinigung und das Rückschalten auf den Berieselungsmodus für die betroffene Behandlungszone. Somit kann praktisch vollautomatisch im laufenden Betrieb eine Innenreinigung einer Behandlungszone vollzogen werden.

Weiterhin kann die zentrale Steuereinheit steuern, zu welcher der Behandlungszonen Z1, Z2, Z3, P1, P2, P3, Z7, Z8, und Z9 das gefilterte Prozesswasser zurück geleitet wird. Insbesondere kann die zentrale Steuereinheit ausgebildet sein, warmes gefiltertes Prozesswasser aus warmen Zonen, beispielsweise aus den Zonen P1, P2 oder P3 zur Innenreinigung der kälteren Zonen Z1, Z2, Z3, Z7, Z8, und Z9 zu verwenden.

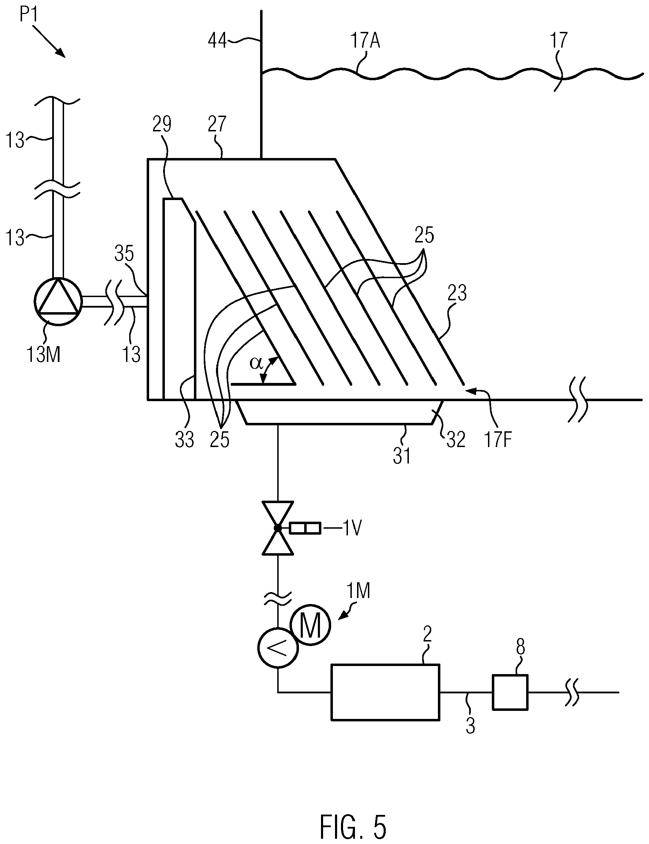

In Figur 4 sind weiterhin für jede der Behandlungszonen Z1, Z2, Z3, P1, P2, P3, Z7, Z8, und Z9 mit ihren jeweiligen Abscheideeinheiten A1, A2, A3, A4, A5, A6, A7, A8 und A9 Leitungen 13 mit Pumpen 13M gezeigt, die aufgefangenes Prozesswasser 17 direkt an die Berieselungsdüsen 15 pumpen können. In der Abscheideeinheit sinken Feststoffe und Partikel typischerweise an den tiefsten Punkt in den Auffangbehältern 19. Eine Entnahme von Prozesswasser 17 oberhalb dieser Punkte kann gewährleisten, dass Prozesswasser wiederverwendet wird, aus dem die Partikel bereits abgesunken sind, das also weniger stark mit Partikeln verschmutzt ist. Eine Weiterbildung dieses Aspekts ist in Figur 5 gezeigt.

In Figur 5 ist am Beispiel einer Behandlungszone, die jeder der in der Figur 4 gezeigten Behandlungszonen Z1, Z2, Z3, P1, P2, P3, Z7, Z8, und Z9 entsprechen kann, eine Weiterbildung für die Abscheideeinheiten A1, A2, A3, A4, A5, A6, A7, A8 und A9 gezeigt. Ausgewählt ist rein beispielhaft die Zone P1 mit der dazugehörigen Abscheideeinheit A4. Gebrauchtes Prozesswasser 17 steht in der Abscheideeinheit A4. Eine Wasserlinie 17A der gesammelten, gebrauchten Prozessflüssigkeit 17 ist eingezeichnet. In einem Auffangbehälter 31, der identisch mit dem Auffangbehälter 19 aus Figur 4 sein kann, wird Sediment 32 aufgefangen. Dieses Sediment wird, wie bereits anhand von Figur 4 erläutert, über ein Ventil 1V und mit Hilfe einer Pumpe 1M der zentralen Filtereinheit 2 zugeführt. Die zentrale Filtereinheit 2 ist identisch mit der in Figur 4 gezeigten zentralen Filtereinheit. Die einzelnen Filtermodule der zentralen Filtereinheit 2 sind in Figur 5 nicht dargestellt. Es soll aber verstanden sein, dass dieselben Filtermodule auch in Figur 5 vorgesehen sind. Über eine Leitung 3 wird das gefilterte Prozesswasser zur Dosierungseinheit 8 geleitet und kann von dort zur weiteren Verwendung, insbesondere zur Innenreinigung der Behandlungszonen verwendet werden, siehe Figur 4.

In Figur 5 ist weiterhin ein Lamellenschrägklärer gezeigt, der Prozesswasser 17 aus der Abscheideeinheit A4 klären soll, bevor es direkt zur erneuten Berieselung verwendet werden kann. In der Figur 5 ist eine Trennwand 23 der Abscheideeinheit A4, die nicht ganz bis zum Boden der Abscheideeinheit A4 führt. Mit dem Pfeil 17F ist eine Fließrichtung oder Strömungsrichtung der gesammelten, gebrauchten Prozessflüssigkeit 17 angedeutet. Dieser Fluss 17F der Prozessflüssigkeit 17 kann durch eine Pumpe 13M erzeugt werden. Der Einsatz einer Absaugvorrichtung oder einer kombinierten Pump- und Absaugvorrichtung (nicht gezeigt) ist ebenfalls möglich. Die Figur 5 zeigt mehrere schräg stehende Lamellen 25 die parallel zueinander angeordnet sind. Der Abstand zwischen den Lamellen 25 typischerweise konstant. Es ist aber ebenso möglich, unterschiedliche Abstände zu wählen oder gruppenweise unterschiedliche Abstände für die Lamellen 25 zu wählen. In Figur 5 sind rein beispielhaft sechs Lamellen 25 dargestellt. Es versteht sich jedoch, dass ebenso eine andere Zahl von Lamellen gewählt werden kann. Die gesammelte Prozessflüssigkeit 17 strömt entlang der Lamellen 25. Über eine Überlaufkante 29 strömt die Prozessflüssigkeit 17 zur Pumpe 13M. Die über die Überlaufkante geströmte Prozessflüssigkeit 17 kann die Abscheideeinheit A4 an der Öffnung 35 wieder verlassen. Von der Öffnung 35 kann die Prozessflüssigkeit durch das Rohr 13 zur Pumpe 13M strömen und von dort wieder zu einer Behandlungszone des Temperierungssystems 100, siehe Figur 4, gelangen. Die Überlaufkante 29 ist rein beispielhaft oberhalb des Endes der Lamellen 25 dargestellt. Es ist jedoch ebenso möglich, das obere Niveau der Überlaufkante 29 entsprechend den oberen Kanten der Lamellen 25 zu wählen. Die Lamellen 25 haben typischerweise die gleiche Größe/Abmessungen. In Figur 5 sind die Lamellen 25 jeweils auf derselben Höhe angebracht. Das bedeutet, dass jeweils das untere und das obere Ende jeder Lamelle denselben Abstand in Bezug auf den Boden der Abscheideeinheit A4 besitzen. Links von den Lamellen 25 ist eine Trennkante 33 vorgesehen, die zusammen mit der Überlaufkante 29 eine Abtrennung der Lamellen 25 gegenüber dem Auslass der Abscheideeinheit 1, d. h. der Öffnung 35 bilden kann. Die Figur 5 gezeigten Lamellen 25 sind unter einem Winkel α zur Horizontalen vorgesehen. Der Winkel α kann beispielsweise 30°< α < 60° betragen, um das Sedimentieren der Partikel 32 unter Einwirkung der Schwerkraft entlang der Flächen der Lamellen 25 zu unterstützen. Somit kann eine noch größere Klärung, also Reinheit des wiederzuverwendenden Prozesswasser erreicht werden.

Die in den Figuren 1 - 5 gezeigten Ausführungsformen können Betriebskosten aus Wasser, Strom und Arbeitszeit, die durch den erzwungenen Betriebsstillstand zur thermischen Desinfektion und zur manuellen Reinigung des Systems anfallen, reduzieren. Prozesswasser kann erneut verwendet werden, sowohl für die Innenreinigung als auch für die Berieselung. Somit können Wartungsintervalle verlängert werden oder in manchen Fällen sogar völlig eingespart werden.

## Patentansprüche

1. Temperierungssystem (100) mit Reinigung der Prozessflüssigkeit, mit
einer Zu- und Abführfördereinrichtung (T1, T2) für Behälter;
mindestens einer Behandlungszone (Z, Z1, Z2, Z3, P1, P2, P3, Z7, Z8, Z9) mit Berieselungsdüsen (15) zum Berieseln der Behälter mit einer Prozessflüssigkeit (17), beispielsweise Wasser, wobei die Behandlungszone (3) eine Abscheideeinheit (A, A1, A2, A3, A4, A5, A6, A7, A8, A9) mit einem Sedimentierungsbereich (19) zum Absetzen von Sediment aus der Prozessflüssigkeit (17) umfasst; und mit
einem Umlaufkreislauf (1, 3, 13) zum Wiederverwenden der Prozessflüssigkeit; **gekennzeichnet durch**
Mittel (1V, 1M) zum zonenweisen Abziehen des Sediments aus dem Sedimentierungsbereich (19) und zum Zuführen des Sediments an eine zentrale Filtereinheit (2);
wobei die zentrale Filtereinheit (2) wenigstens ein Filtermodul (11) zum Filtern von Feststoffen aus dem zugeführten Sediment umfasst, so dass gefilterte Prozessflüssigkeit erhalten wird; und durch
Mittel (3, 3V) zum Rückleiten der gefilterten Prozessflüssigkeit an eine oder mehrere Behandlungszonen (Z, Z1, Z2, Z3, P1, P2, P3, Z7, Z8, Z9).

2. System (100) gemäß Anspruch 1, wobei die Mittel zum zonenweisen Abziehen des Sediments aus dem Sedimentierungsbereich (19) ausgebildet sind, eine Wirbelströmung auszubilden derart, dass das Sediment aus dem Sedimentierungsbereich (19) abgezogen werden kann.

3. System (100) gemäß Anspruch 1 oder 2, wobei die zentrale Filtereinheit (2) ein weiteres Filtermodul (4) zum Filtern von Schwebstoffen umfasst.

4. System (100) gemäß wenigstens einem der Ansprüche 1 - 3, wobei die zentrale Filtereinheit (2) ein weiteres Filtermodul (5) umfasst, welches ausgebildet ist, Mikrofiltration und/oder Ultrafiltration und/oder Nanofiltration und/oder Umkehr-Osmosefiltration durchzuführen.

5. System (100) gemäß wenigstens einem der Ansprüche 1 - 4, wobei die zentrale Filtereinheit (2) ein weiteres Filtermodul (7) zum Bestrahlen der gefilterten Prozessflüssigkeit mit UV-Strahlung umfasst.

6. System (100) gemäß wenigstens einem der Ansprüche 1 - 5, wobei das System (100) eine Dosierungseinheit (8) umfasst, die ausgebildet ist, Biozid zu der von der zentralen Filtereinheit (2) gefilterten Prozessflüssigkeit hinzu zu dosieren.

7. System (100) gemäß wenigstens einem der Ansprüche 1 - 6, wobei eine oder mehrere oder alle der Behandlungszonen (Z, Z1, Z2, Z3, P1, P2, P3, Z7, Z8, Z9) jeweils ein Innenreinigungsmodul mit einer oder mehreren Düseneinrichtungen (6.1, 6.2, 6.3) umfasst, welche ausgebildet sind, einen oder mehrere Innenbereiche der Behandlungszonen (Z, Z1, Z2, Z3, P1, P2, P3, Z7, Z8, Z9) mit gefilterter Prozessflüssigkeit zu reinigen.

8. System (100) gemäß Anspruch 7, wobei wenigstens eine der Düseneinrichtungen (6.1) ausgebildet ist, die Decke oberhalb der Berieselungsdüsen (15) mit gefilterter Prozessflüssigkeit abzuspritzen.

9. System (100) gemäß Anspruch 7 oder 8, wobei in den Behandlungszonen (Z, Z1, Z2, Z3, P1, P2, P3, Z7, Z8, Z9) die zu berieselnden Behälter auf mehreren übereinander angeordneten Fördereinrichtungen (T1, T2) geführt werden und wenigstens eine der Düseneinrichtungen (6.2) zwischen zwei übereinander angeordneten Fördereinrichtungen (T1, T2) angeordnet ist derart, dass zwischen den Fördereinrichtungen (T1, T2) angeordnete Flächen gereinigt werden können.

10. System (100) gemäß wenigstens einem der Ansprüche 7 - 9, wobei wenigstens eine der Düseneinrichtungen (6.3) so angeordnet ist, dass Flächen, die im Betrieb unter der Wasserlinie liegen, abschwallbar sind.

11. System (100) gemäß wenigstens einem der Ansprüche 7 - 10, wobei die Düseneinrichtungen (6.1, 6.2, 6.3) Rotationsdüsen umfassen, die um 360° drehbar angeordnet sind.

12. System (100) gemäß wenigstens einem der Ansprüche 1 - 11, ferner mit einer Steuereinheit, die ausgebildet, die Mittel zum zonenweisen Abziehen des Sediments aus dem Sedimentierungsbereich (19) und zum Zuführen des Sediments an die zentrale Filtereinheit (2) zu steuern.

13. System (100) gemäß Anspruch 12, wobei die Steuereinheit ausgebildet ist, die Temperatur der Prozessflüssigkeit der Behandlungszonen (Z, Z1, Z2, Z3, P1, P2, P3, Z7, Z8, Z9) zu messen und ausgebildet ist, gefilterte Prozessflüssigkeit aus wenigstens einer Behandlungszone (Z, Z1, Z2, Z3, P1, P2, P3, Z7, Z8, Z9) mit einer höheren Temperatur der Prozessflüssigkeit zur Innenreinigung einer Behandlungszone (Z, Z1, Z2, Z3, P1, P2, P3, Z7, Z8, Z9) mit kälterer Prozessflüssigkeit zu verwenden.

14. System (100) gemäß wenigstens einem der Ansprüche 1 - 13, ferner wobei die Abscheideeinheit (A, A1, A2, A3, A4, A5, A6, A7, A8, A9) in der Behandlungszone (Z, Z1, Z2, Z3, P1, P2, P3, Z7, Z8, Z9) ferner eine Pumpe (13M) und einen unter der Flüssigkeitsoberfläche angeordneten Lamellenschrägklärer mit mehreren parallelen, schräg angeordneten Lamellen (25) umfasst, wobei die Pumpe (13M) die Prozessflüssigkeit (17) entlang der Lamellen (25) pumpt; wobei insbesondere die Pumpe (13M) die Prozessflüssigkeit (25) über den tiefsten Punkt einer Behandlungszone (Z, Z1, Z2, Z3, P1, P2, P3, Z7, Z8, Z9) pumpt, so dass sich Sediment absetzen kann.

15. System (100) gemäß wenigstens einem der Ansprüche 1 - 14, wobei das Temperierungssystem ein Pasteurisationssystem, ein Wärmesystem oder ein Kühlsystem ist.
